Europäisches Patentamt

European Patent Office

Office européen des brevets

(19)

(11) Numéro de publication : **0 469 110 B1**

(12) **FASCICULE DE BREVET EUROPEEN**

(45) Date de publication du fascicule du brevet :
27.07.94 Bulletin 94/30

(51) Int. Cl.⁵ : **B01J 13/12, A61K 9/52**

(21) Numéro de dépôt : **91903868.7**

(22) Date de dépôt : **18.02.91**

(86) Numéro de dépôt international :
**PCT/EP91/00307**

(87) Numéro de publication internationale :
**WO 91/12882 05.09.91 Gazette 91/21**

(54) **MICROSPHERES POUR LA LIBERATION CONTROLEE DES SUBSTANCES HYDROSOLUBLES ET PROCEDE DE PREPARATION.**

(30) Priorité : **22.02.90 FR 9002189**

(43) Date de publication de la demande :
**05.02.92 Bulletin 92/06**

(45) Mention de la délivrance du brevet :
**27.07.94 Bulletin 94/30**

(84) Etats contractants désignés :
**AT BE CH DE DK ES FR GB GR IT LI LU NL SE**

(56) Documents cités :
**EP-A- 0 102 265**
**DE-A- 2 237 545**

(73) Titulaire : **TEVA PHARMACEUTICAL INDUSTRIES LTD.**
**Science Based Industrial Park**
**Har Hahotzvim**
**Jerusalem (IL)**

(72) Inventeur : **WANTIER, Henri**
**Rue de Baisieux, 13**
**B-7370 ELOUGES (BE)**
Inventeur : **MATHIEU, Fabienne**
**86, rue du Cheval-Godet**
**B-1400 Nivelles (BE)**
Inventeur : **BAUDRIHAYE, Marc**
**15, avenue les Libellules**
**B-1410 Waterloo (BE)**
Inventeur : **DELACROIX, Dominique**
**11, avenue des Ramiers**
**B-1950 Kraainem (BE)**

(74) Mandataire : **Schrimpf, Robert et al**
**Cabinet Regimbeau**
**26, Avenue Kléber**
**F-75116 Paris (FR)**

## Description

La présente invention concerne des microparticules du type microsphères constituées d'une matrice de polymère biocompatible et biodégradable au sein de laquelle est dispersée une substance hydrosoluble notamment un polypeptide, le polymère contrôlant la cinétique de relargage de ladite substance, de sorte que ladite substance est progressivement relarguée sur une période de temps déterminée lorsque les microparticules sont placées dans un environnement aqueux, notamment de type physiologique.

La présente invention concerne également un procédé de préparation desdites microparticules.

De telles microsphères ont été initiallement développées pour la contraception avec les hormones stéroïdes (voir notamment US 3 773 919).

Une difficulté des inventions dans ce domaine est la nécessité de déterminer les polymères présentant les propriétés définies en fonction du type de principe actif à incorporer compte tenu d'une part de sa structure et ses propriétés chimiques, et d'autre part de la cinétique de relargage recherché. Il convient également de déterminer pour un type de substance donné, la vitesse de libération et le taux de charge assurant une couverture thérapeutique optimum pour une période choisie lorsque ladite substance est un médicament.

De plus, certaines limitations peuvent intervenir dans le procédé de préparation, au niveau de la technique d'incorporation du médicament dans le polymère qui dépend du couple polymère médicament, notamment de l'existence éventuellement d'un solvant commun des partenaires, l'existence de propriétés thermiques de ceux-ci, de leur miscibilité, etc. En outre, cette incorporation ne doit pas altérer les propriétés du principe actif.

A l'origine, le but de la présente invention était la préparation de microsphères contenant un polypeptide hydrosoluble et notamment de la calcitonine, et relargant celle-ci de façon progressive et constante pendant une période d'au moins 8 jours et même de 30 jours pour la calcitonine une fois injectée à l'homme.

L'incorporation de principes actifs dans des biomatériaux polymères sous forme de microsphères se fait par les techniques dites de microencapsulation. La microencapsulation regroupe l'ensemble des techniques permettant l'obtention de particules individualisées du type microsphère dont la taille s'échelonne entre 1 et 250 µm.

Les procédés de microencapsulation sont classés traditionnellement en deux groupes.

### 1. La microencapsulation dite par coacervation ou par séparation de phase

Dans un premier temps, le produit à encapsuler est dispersé dans la solution d'un polymère destiné à former par la suite la matrice des microcapsules. Dans un deuxième temps, la coacervation du polymère est induite par une modification physico-chimique du milieu réactionnel, notamment à l'aide d'un agent inducteur de séparation de phase. Dans un troisième temps, des goutellettes coacervats formées enrobant le matériau à encapsuler sont stabilisées et solidifiées à l'aide d'un non-solvant du polymère, par l'exemple l'heptane.

L'inconvénient majeur de cette méthode est la mise en oeuvre de grande quantité de solvants avec outre des contraintes de coût, des problèmes de toxicité liés aux solvants utilisés tels que l'heptane. Les techniques par coacervation mettant en oeuvre de l'heptane ne permettent pas en effet d'éliminer complètement celui-ci. On observe une quantité importante de solvants résiduels de l'ordre de 5 à 10 % d'heptane dans les microsphères.

Indépendamment de ce qui précède, il a été également constaté que l'on obtenait souvent des agrégats de microsphères entrainant une perte importante de rendement dans la production de ces microsphères par cette méthode et nécessitant quelques fois le rejet total de certains lots devenus de la sorte inutilisable. La tendance des microsphères à s'agréger provoque des difficultés supplémentaires au moment de la mise en suspension des microsphères pour l'injection, dans le cas de microsphères injectables.

Un autre inconvénient de la technique par séparation de phase est la répartition inhomogène de la substance active dans les microsphères avec un relargage irrégulier, et en général une première phase de relargage accéléré ("burst effect"). Ceci s'observe particulièrement lorsque la substance active est mise en suspension dans la solution de polymère, notamment parce qu'elle n'est pas soluble dans le solvant du polymère. Ceci s'applique d'une manière générale, par exemple, pour des polypeptides.

### 2. La microencapsulation par évaporation de solvant

Le procédé dit par évaporation de solvant (ou encore par émulsion/ évaporation) conduit également à l'obtention de microsphères. Le principe actif à encapsuler est traditionnellement dispersé dans une solution de polymère dans un solvant organique volatile. Cette phase est émulsionnée à l'aide d'un agent surfactif dans un milieu dispersant non miscible (eau ou huile minérale). Sous agitation, le solvant organique s'évapore. A l'issu de l'évaporation, les microsphères sont récupérées par filtration ou centrifugation.

Les avantages de la technique par émulsion/évaporation sont l'absence de solvants toxiques tels que l'heptane, et l'absence d'agglomération des microsphères.

Cette technique par émulsion/évaporation est une technique plus simple, plus souple et plus facilement industrialisable que la technique par séparation de phase ou coacervation. Elle permet de mettre en oeuvre des quantités réduites de solvant.

Elle offre une grande liberté d'action sur différents paramètres tels que la taille des microsphères, la vitesse de relargage, le taux de charge de la substance active.

Traditionnellement, cette technique s'adresse en priorité à l'encapsulation de substances lipophiles, telles que les stéroïdes, les nitrosourés (EP-A- 0 102 265).

La microencapsulation de principes actifs hydrophiles requière l'utilisation d'une phase dispersante apolaire telle qu'une huile minérale. On utilise classiquement les systèmes acétone/parafine. Toutefois, les taux d'incorporation du principe actif hydrophile dans les microsphères par rapport aux quantités engagées dans le procédé sont assez faibles, et d'autre part ce système implique une limitation quant aux types de polymères utilisables dans la mesure où il requiert que le polymère soit soluble dans l'acétone, ce qui est le cas des polymères d'acide lactique, mais ce qui n'est pas le cas des copolymères d'acides lactique et glycolique.

Cette technique par émulsion/évaporation est donc reconnue traditionnellement comme inadaptée pour les peptides hydrosolubles et pour toutes substances hydrosolubles

La méthode par séparation de phase ou coacervation est donc la méthode retenue comme étant la mieux adaptée aux substances hydrosolubles, notamment aux polypeptides hydrosolubles.

Pour tenter de pallier aux inconvénients de la méthode par émulsion/évaporation, une méthode par double émulsion (eau/huile/eau) a été décrite dans la demande de brevet européen EP 190 833. Elle est cependant complexe, relativement difficile à industrialiser et ne permet pas d'obtenir la cinétique de relargage souhaitée selon la présente invention.

Le but de la présente invention est de fournir des microparticules de type microsphères pour la libération progressive d'une substance active hydrosoluble tel qu'un médicament, notamment d'un polypeptide hydrosoluble, dispersé dans une matrice de polymère biocompatible et biodégradable permettant un relargage de la substance active sur notamment une période d'au moins 8 jours, et ce de façon régulière avec notamment l'absence d'une première phase de relargage accéléré ("burst effect").

Plus particulièrement, un autre but de la présente invention est de permettre la libération progressive de calcitonine sur une période d'environ 30 jours dans les mêmes conditions que mentionné ci-dessus.

Un autre but est fournir un procédé de préparation de ces microsphères économiquement et industriellement acceptables avec notamment un taux d'incorporation de la substance active à incorporer sous forme de microsphère suffisamment élevé.

Un autre but est de fournir des microsphères et un procédé de préparation tels que le taux résiduel de solvants, notamment de solvants toxiques, soit très faible.

On a découvert selon la présente invention un nouveau procédé permettant d'adapter la technique par émulsion/évaporation aux substances hydrosolubles et notamment aux polypeptides hydrosolubles avec un taux d'incorporation de la substance dans les microsphères supérieur à 90 % et un taux de solvant résiduel très faible.

On entend dans la présente demande par "taux d'incorporation", le taux de substance qui se retrouve dans les microsphères en fin de procédé par rapport à la quantité de substance initiallement engagée dans le procédé.

Contrairement aux méthodes décrites dans la littérature dans laquelle la substance active à incorporer est un peptide qui est mis en suspension micro-particulaire dans la solution du polymère, selon l'invention, la substance active peptidique est tout d'abord solubilisé(e) dans un solvant approprié dont les caractéristiques seront indiquées ci-après.

La présente invention a donc pour objet un procédé de préparation des microsphères d'une substance hydrosoluble, notamment d'un polypeptide hydrosoluble et d'un polymère biocompatible et biodégradable contrôlant la cinétique de libération de ladite substance selon lequel :

a) le polymère est dissous dans un premier solvant organique volatile et non miscible à l'eau,

b) la substance hydrosoluble est dissoute séparément dans un second solvant qui est miscible au premier solvant, qui est également solvant du polymère, et qui est miscible à l'eau (ledit second solvant constitue donc en fait un tiers solvant entre l'eau et le premier solvant, qui est à la fois solvant du polymère et solvant de la substance hydrosoluble), la proportion en second solvant par rapport au premier solvant allant de 5/95 à 70/30 en volume.

c) on mélange la solution du polymère obtenue à l'étape a) et la solution de la substance active obtenue à l'étape b),

d) on obtient une phase organique du polymère et de la substance active qui est ensuite émulsionnée dans un milieu non miscible dispersant, consistant en une phase aqueuse contenant un agent émulsifiant,

e) les deux solvants sont ensuite éliminés des microsphères en formation, sous agitation, le premier solvant étant éliminé par évaporation, le second solvant ainsi qu'une partie du premier solvant qui lui est miscible passant dans la phase aqueuse par un mécanisme de séparation de phase,

f) après élimination des solvants, les microsphères formées sont récupérées éventuellement après lavage à l'eau et tamisage.

En outre, les microsphères sont ensuite de préférence traitées de manière à assurer leur conservation.

On entend par solvant organique volatile un solvant facilement évaporable, c'est-à-dire évaporable sous agitation à température ambiante et pression réduite sous aspiration d'air.

Le deuxième solvant est mis en proportion telle par rapport au premier solvant qu'il n'y ait pas de précipitation du polymère dans la phase aqueuse. En effet, si la quantité dudit second solvant est trop importante. l'émulsion n'est plus obtenue, la phase polymère et la phase aqueuse deviennent miscibles et il y a précipitation du polymère dans la phase aqueuse. L'émulsion reste possible avec des rapports (second solvant/ premier solvant) allant jusqu'à 70/30 en volume. La proportion minimale en second solvant par rapport au premier solvant est bien entendu liée au taux de charge que l'on souhaite obtenir en ce qui concerne la substance active dans la microsphère. On peut donner comme valeur minimale la proportion 5/95.

On peut citer de façon non limitative, à titre de premier solvant, le chloroforme ou le dichlorométhane et à titre de second solvant, le diméthylacétamide DMA, le tétrahydrofurane THF, le dioxane, le DMSO (diméthylsulfoxide) ou le DMF (diméthylformamide).

Dans un mode préféré de réalisation du procédé selon l'invention, le polymère est dissous dans le dichlorométhane et la substance hydrosoluble est dissoute dans le diméthylacétamide (DMA).

L'utilisation du tiers solvant, qui est à la fois solvant de la substance hydrosoluble et miscible au solvant du polymère, assure une répartition homogène de la substance active dans la phase polymère. Toutefois, l'intérêt d'utilisation du tiers solvant comme on l'a vu ne se réduit pas à la simple solubilisation de la substance active. Compte tenu de sa miscibilité à la phase aqueuse, il contribue également au processus d'incorporation de la substance active dans la microsphère.

En effet, contrairement à la méthode classique par émulsion/ évaporation où le durcissement de la microsphère se fait uniquement par simple et lente évaporation du solvant du polymère tel que $CH_2Cl_2$, dans la méthode selon la présente invention le phénomène est lié simultanément à l'évaporation dudit premier solvant tel que $CH_2Cl_2$ et à une séparation de phase rapide dudit second solvant tel que le DMA vers la phase aqueuse. De par la miscibilité des deux solvants, une partie du premier solvant accompagne également le second vers la phase aqueuse ce qui renforce et accéllère considérablement son extraction. La méthode associe ainsi les processus d'évaporation et de séparation de phase en modifiant fortement la cinétique d'extraction des solvants par rapport à une méthode d'émulsion/évaporation traditionnelle. La cinétique du processus d'incorporation est donc modifiée ce qui contribue avec l'homogénéité de répartition au taux élevé d'incorporation observé comme il sera explicité par la suite.

Un des avantages du procédé selon l'invention est de fournir des microsphères dont la teneur résiduelle en solvant toxique, tel que le dichlorométhane, est très faible (notamment inférieure à 1,5 %).

Dans un mode perfectionné de mise en oeuvre du procédé selon l'invention, notamment intéressant lorsque ledit premier solvant du polymère est un solvant toxique, le taux de solvant résiduel peut être réduit si la phase dispersante aqueuse comporte un troisième solvant non toxique miscible à l'eau et miscible audit premier solvant du polymère. Ce troisième solvant est ajouté à la phase dispersante aqueuse de l'émulsion avant le début de formation de cette émulsion. Cet ajout d'un tel troisième solvant permet de favoriser l'élimination du premier solvant du polymère par une meilleure extraction de ce dernier vers la phase aqueuse à laquelle il est sinon non miscible. Le taux résiduel en solvant du polymère dans les microsphères s'en trouve par là réduit.

Ledit troisième solvant est ajouté à la phase aqueuse dans des proportions rendant toujours possible l'émulsion et la mise en solution de l'agent émulsifiant dans cette même phase aqueuse.

Lorsque le solvant du polymère est le dichlorométhane on utilise avantageusement l'éthanol comme dit troisième solvant dans une proportion allant jusqu'à 20 % en volume de la phase aqueuse. Le taux résiduel en dichlorométhane dans les microsphères s'en trouve réduit considérablement, par exemple à 0,2 % par rapport au poids sec des microsphères.

Dans un mode de réalisation particulier de l'invention, la substance hydrosoluble est un polypeptide ou un de ses sels pharmaceutiquement acceptable et le polymère est un polymère résultant de la condensation de monomères choisis parmi les acides alpha-hydroxycarboxylique et les lactones. De préférence, le polymère est un copolymère d'acide lactique (acide alpha-hydroxypropionique) et d'acide glycolique (acide alpha-hydroxyacétique) ci-après abrégé PLGA, dans des proportions variables entre les unités d'acide lactique et d'acide glycolique, ou un mélange de tels polymères.

Ces polymères sont bien connus dans l'état de la technique. Ils sont connus pour générer des produits naturellement présents et métabolisés dans l'organisme et sont donc considérés comme biodégradables et dépourvus de toxicité significative dans les conditions usuelles d'utilisation. Ils sont en outre solubles dans le dichlorométhane.

Le DMA présente toutes les caractéristiques requises à titre de second solvant dans la mesure où les polymères cités ci-dessus et en particulier les copolymères d'acides lactique et glycolique sont solubles dans celui-ci. Le DMA n'est pas évaporable dans les conditions aisées contrairement au dichlorométhane. En revanche, il peut être extrait par la phase aqueuse à laquelle il est miscible, éventuellement au cours de l'évaporation du dichlorométhane.

Dans un mode particulier de réalisation de l'invention, on utilise un copolymère d'acide lactique et glycolique (PLGA) qui comprend de préférence une proportion entre les unités d'acide lactique et d'acide glycolique allant respectivement de 40:60 à 75:25.

La masse moléculaire du polymère sera de préférence compris entre 20 000 et 100 000. Les polypeptides hydrosolubles selon l'invention ont une masse moléculaire habituellement comprise entre 200 et 100 000.

Ainsi le procédé selon l'invention est applicable aux polypeptides les plus divers tels que des hormones, des cytokines, des facteurs de croissance, des neuropeptides, des facteurs de coagulation, des enzymes, des anti-enzymes, des récepteurs solubles ou des dérivés biologiquement actifs de ces substances. On peut citer en particulier de manière non limitative, les hormones de croissance humaine (HGH), bovine (BGH), porcine (PGH), ovine, etc.; les somatomédines, IGF-1, IGF-2, et les insulines ; la LH-RH et ses analogues, le GH-RP et ses analogues ; la somatostatine et ses analogues ; l'ACTH, l'ADH, la PTH, la PTH I-34, le CRH, le GH-RH, l'inhibine, l'activine, la motiline, la relaxine, l'ANF, les endothélines, leurs dérivés et analogues ; l'IL1, L'IL2, l'IL3, l'IL4, l'IL5, l'IL6, l'IL7, l'IL8, l'IL9, le G-CSF, le GM-CSF, le M-CSF, les interférons alpha, bêta et gamma, les TNF alpha et bêta, les TGF alpha et bêta, le PDGF, l'EGF, le FGF, les osteogénines, leurs analogues et dérivés; le TPA, le facteur VIII, le facteur de von Willebrandt, l'hirudine, l'echistatine, la bitisatine, l'alpha-1 antitrypsine, la superoxide dismutase, le CD4 soluble et leurs analogues et dérivés ; les peptides dérivés ou analogues des molécules d'adhésion, des récepteurs lymphocytaires ou des composants du complexe majeur d'histocompatibilité ; et les calcitonines qu'elles soient originaires de l'homme, du porc, du saumon, de l'anguille, ou d'une autre espèce, ainsi que leurs analogues et dérivés.

Selon le procédé de l'invention, le taux de charge en substance dans le polymère peut s'élever de 0,01 à 40 %. On entend par taux de charge, le rapport en poids de la substance sur le poids du polymère dans la microsphère. En général, en fonction de la couverture thérapeutique recherchée, ce taux de charge sera de préférence de 1 à 10 %.

Ainsi, s'agissant de la calcitonine, sa solubilité dans le DMA qui est de 50 mg/ml permet d'envisager un taux de charge allant jusqu'à 31,5 % de calcitonine de saumon dans des microsphères de PLGA 50:50 compte tenu de la proportion maximale $DMA/CH_2 Cl_2$ de 70/30 que l'on peut utiliser. Toutefois, la meilleure couverture thérapeutique est obtenue avec seulement un taux de charge de 1 à 2 % s'agissant de la calcitonine de saumon, et de 10 % s'agissant de la calcitonine humaine qui est moins active. Il apparaît donc que le procédé selon l'invention n'apporte aucune limitation dans la pratique quant au taux de charge de la substance dans la microsphère.

On entend dans la présente demande par polypeptide hydrosoluble une solubilité d'au moins 0,1 mg/ml dans l'eau.

Lorsque la solubilité du polypeptide est trop faible pour le taux de charge que l'on souhaite obtenir dans la microsphère compte tenu de la couverture thérapeutique visée, on peut utiliser comme second solvant un mélange de DMA et d'eau, la quantité d'eau pouvant aller en proportion en volume par rapport au DMA jusqu'à 50 %.

Selon l'invention, il peut être rajouté en même temps que la substance active dans la phase polymère une substance ballast afin de faciliter le relargage en accélérant et en régularisant la cinétique de relargage. Ce ballast peut être soit mis en solution, soit mis en suspension dans la phase polymère. Les ballasts les plus divers peuvent être utilisés tels que des protéines comme la HSA, la gélatine, des sucres, des polysaccharides, des acides aminés, ou des polypeptides. Le taux de charge en ballast peut atteindre 25 %.

Dans le procédé selon l'invention, de préférence l'émulsification se fait sous agitation violente de manière à obtenir des microparticules de taille inférieure à 200 μ de préférence entre 25 et 200 μ. Ainsi, on obtient des microsphères injectables lorsqu'elles sont formulées dans un soluté injectable.

La taille des microsphères dépend en effet de la vigueur de l'agitation et notamment de la vitesse imposée à l'hélice lors de l'émulsion (si elle augmente la taille diminue). Elle dépend également de la concentration d'émulsifiant dans la phase aqueuse (si elle augmente la taille diminue), et enfin bien sûr de la taille des mailles des tamis. On obtient aisément une répartition maximale de la taille moyenne des microsphères entre 25 μ et 200 μ de manière reproductible en jouant sur les paramètres précités.

A titre d'agent émulsifiant présent dans la phase aqueuse, on peut citer de façon non limitative la gélatine, la carboxyméthylcellulose, la méthylcellulose, l'hydroxypropylcellulose, l'hydroxyéthylcellulose, l'alcool poly-vinylique (PVA) ou encore des détergents non ioniques tels que le tween 80 ou des détergents ioniques.

La concentration de l'émulsifiant dans la phase aqueuse influence la taille des microsphères. Celle-ci augmente quand la concentration en émulsifiant diminue. Selon l'invention, la concentration en émulsifiant peut aller de 0,1 à 10 % (poids/volume) dans la phase aqueuse.

Dans un mode de réalisation particulier, on a utilisé comme agent émulsifiant la gélatine qui est particulièrement bien acceptée comme produit injectable dans l'organisme.

Le procédé selon l'invention offre les avantages habituels d'une technique d'émulsion/évaporation par rapport à la technique de séparation de phase ou coacervation :
- absence de solvant toxique autre que le solvant du polymère,
- absence d'agglomération de microsphères (industrialisation et injection plus aisée),
- absence de manipulation de grande quantité de solvant lors de la production industrielle,
- possibilité d'agir sur différents paramètres pour moduler aisément la cinétique de relargage en fonction de l'application thérapeutique concernée : les caractéristiques du polymère, la présence de ballast, la taille des microsphères, le taux de charge en substance hydrosoluble.

Les autres avantages propres à l'utilisation d'un tiers solvant pour solubiliser la substance hydrosoluble sont:
- un taux d'incorporation de la substance pouvant être supérieur à 90 %,
- la répartition régulière et homogène de la substance hydrosoluble solubilisée dans le polymère, et dans la microsphère finale,
- la possibilité d'atteindre des taux de charge en substance hydrosoluble importants tout en conservant un taux d'incorporation élevé,
- une réduction du taux résiduel du solvant du polymère dans le produit final,
- l'absence ou la réduction d'effet "burst" lors de la cinétique de relargage de la substance.

La présente invention a donc également pour objet des microparticules de type microsphères pour la libération progressive d'une substance active hydrosoluble telle qu'un médicament, notamment d'un polypeptide hydrosoluble obtenu par le procédé selon l'invention. Dans ces microsphères, la substance active est répartie de façon régulière au sein d'une matrice d'un polymère biocompatible et biodégradable soluble dans un solvant organique non miscible dans l'eau permettant un relargage de ladite substance active sur une période d'au moins 8 jours, et ce de façon régulière avec notamment l'absence d'une première phase de relargage accéléré.

Selon l'invention, les microsphères présentent un taux résiduel en solvant inférieur à 1,5 % de préférence inférieur à 0,5 % (w/w) et notamment sont dépourvues d'heptane. En effet le taux en DMA est inférieur à 0,5 %, et en général même de l'ordre de 0,1 % (w/w) et le taux en $CH_2Cl_2$ est en général inférieur à 0,5 %, et même souvent inférieur à 0.2 % (w/w). En conséquence, une des caractéristiques des microsphères selon la présente invention est d'avoir un taux en solvant toxique résiduel inférieur à 0,5 96, de préférence inférieur à 0,2 96 en poids par rapport au poids sec des microsphères.

Le polymère sera avantageusement un polymère soluble dans le dichlorométhane, notamment un copolymère d'acides lactique et glycolique.

Les microsphères obtenues selon l'invention se caractérisent outre par la répartition homogène de la substance active qui se manifeste par la forte réduction de l'effet burst, par une faible tendance à s'agglomérer ce qui les distinguent des microsphères obtenues par coacervation habituellement décrit pour l'encapsulation des peptides, et par des taux résiduels en solvants fortement réduits.

D'autres caractéristiques et avantages de la présente invention apparaîtront à la lumière des exemples qui vont suivre.

## EXEMPLE 1 : PREPARATION DE MICROSPHERE DE CALCITONINE DANS UNE MATRICE DE COPOLYMERE D'ACIDES LACTIQUE ET GLYCOLIQUE

On met en solution 5 g de PLGA 50/50 (copolymère d'acides lactique et glycolique : 50/50) dans 40 g de dichlorométhane $CH_2Cl_2$.

On met en solution 5 mg de calcitonine de saumon dans 5 ml de diméthylacétamide (DMA). On mélange le peptide solubilisé à la phase polymère. On ajoute à ce mélange 150 ml de tampon phosphate 10 mM à pH 8 contenant 4 % (poids/volume) de gélatine.

On réalise une émulsion en mélangeant la phase polymère/ calcitonine avec la phase aqueuse contenant la gélatine à l'aide d'une hélice tournant à 1500 tours/minute pendant 1 minute.

On procède à l'évaporation du solvant du polymère $CH_2Cl_2$ en maintenant une agitation de 500 tours/minute dans le mélange émulsionné. Parallèlement s'effectue l'extraction du DMA vers la phase aqueuse. Cette évaporation se fait à température ambiante avec aspiration d'air jusqu'à évaporation complète du solvant.

Le mélange émulsionné est ensuite dilué deux fois avec de l'eau et agité 1 heure à 37°C.

Les microsphères ainsi obtenues sont ensuite tamisées dans l'eau afin de sélectionner celles ayant une taille adéquate pour être injectées, c'est-à-dire comprise entre 25 et 200 μ.

Les microsphères tamisées sont ensuite séchées sous vide à température ambiante.

Après séchage, les microsphères sont bien individualisées et se remettent aisément en suspension dans un milieu physiologique pour injection. L'examen microscopique confirme l'homogénéité et la totale absence d'agglomération des microsphères.

Les taux en solvants résiduels dans les microsphères sont :
- solvant du polymère
  $CH_2Cl_2$ : inférieur à 0,5 % par rapport au poids sec des microsphères,
- tiers solvant
  DMA : inférieur à 0,1 % par rapport au poids sec des microsphères

## EXEMPLE 2 : MODIFICATION DE L'EXTRACTION DU SOLVANT DU POLYMERE PAR L'USAGE DU TIERS SOLVANT

Cet exemple illustre l'amélioration de l'élimination du premier solvant ($CH_2Cl_2$) par le mécanisme de séparation de phases grâce à la présence du second solvant (DMA).

Matériel

Phase polymère :
- PLGA 50/50 $\eta$ = 0,8 : 2,5 g
- CT saumon        : 5 mg
  taux de charge de 0,2 %/PLGA.

| A | B |
|---|---|
| Volume $CH_2Cl_2$/ Volume DMA = 9/1 <br> $CH_2Cl_2$ = 20,25 g <br> DMA = 2,25 ml | Volume $CH_2Cl_2$/ Volume DMA = 7/3 <br> $CH_2Cl_2$ = 15,75 g <br> DMA = 6,75 ml |

Phase aqueuse :
- Tampon phosphate 10 mM pH 8 avec 4 % de gélatine comme émulsifiant : 75 ml.

Méthode

- Le peptide est solubilisé dans le volume de DMA intervenant dans la réaction.
- Cette solution est ensuite ajoutée au PLGA solubilisé dans le $CH_2Cl_2$.
- La phase aqueuse est mélangée à cette phase PLGA grâce à une hélice tournant à 1500 t/min pendant 1 minute.
- Une agitation de 500 t/min est maintenue pendant 12 heures à température ambiante avec aspiration d'air à la surface du milieu réactionnel.
- Les microsphères sont alors tamisées et lavées à l'eau.
- Elles sont ensuite séchées dans une étuve à température ambiante, sous vide.

Résultats

Le taux résiduel en $CH_2Cl_2$ dans ces microsphères est mesuré par chromatographie gazeuse.

|  | A | B |
|---|---|---|
| Volume $CH_2Cl_2$/Volume DMA | 9/1 | 7/3 |
| Taux résiduel en $CH_2Cl_2$ (%) (poids $CH_2Cl_2$/poids microsphères) | 2,58 % | 0,44 % |

**EXEMPLE 3 : TAUX D'INCORPORATION DE CALCITONINE ET EFFET "BURST"**

Les microsphères obtenues par le procédé selon l'invention se caractérisent par une diminution du "burst effect" et un taux d'incorporation très amélioré par rapport au procédé classique par émulsion/évaporation.

Le tableau ci-après reproduit les résultats des préparations obtenues avec la méthode classique par émulsion/évaporation comparée à la méthode selon l'invention pour différents polymères.

La présence d'un effet "burst" reflète essentiellement inhomogénéité de la répartition du principe actif dans la matrice de PLGA.

Dans la méthode "classique" par émulsion/évaporation suivie et la méthode selon l'invention, on a utilisé 2,5 g de polymère mis en solution dans 20 g de $CH_2Cl_2$. On a utilisé 5 mg de calcitonine de saumon et 10 mg de gélatine à titre de ballast.

Dans la méthode "classique", la calcitonine ainsi que le ballast sont mis en suspension dans la phase polymère. Dans la méthode selon l'invention, la calcitonine est dissoute dans 2,5 ml de DMA auquel est ajouté le ballast sous forme d'une solution aqueuse de gélatine à 5 % (0,2 ml). Ce mélange calcitonine-ballast-DMA est alors ajouté à la phase polymère de façon à former une seule phase homogène.

Dans les méthodes, la phase aqueuse consiste en 75 ml de gélatine 4 % dans l'eau (poids/volume). Les deux phases sont émulsionnées par agitation d'une hélice à 1500 tours/minute pendant 1 minute. Une agitation de 500 tours/minutes est maintenue d'une part pour faciliter l'évaporation du $CH_2Cl_2$, et d'autre part dans la méthode selon l'invention, de permettre au DMA de s'extraire dans la phase aqueuse, dans les deux cas, les microsphères sont lavées à l'eau et récoltées sur des tamis.

| | Méthode "classique" par émulsion/ évaporation | Méthode d'émulsion/évaporation selon l'invention |
|---|---|---|
| Répartition de peptide dans la phase PLGA : | par mise en suspension | par mise en solution grâce à un tiers solvant (DMA) |
| Taux d'incorporation de la calcitonine dans les micro-sphères de PLGA avec : | | |
| exemple 3-1 : PLGA 50/50 de Mw = 50 000 n = 0,5 | 29,8 % | 79,4 % |
| exemple 3-2 : PLGA 50/50 de Mw = 91 000 n = 0,7 | 39,7 % | 83,8 % |
| exemple 3-3 : PLGA 50/50 de Mw = 115 000 n = 0,8 | 52,6 % | 92,4 % |
| Effet "Burst" observé après le premier jour de relargage in vitro | | |
| exemple 3-1 : | 12 % | 5 % |
| exemple 3-2 : | 30 % | 3 % |
| exemple 3-3 : | 11 % | 5 % |

La viscosité du polymère est représentée par la lettre n. Elle est reliée à la masse moléculaire Mw par la relation de Kuhn-Mark-Houwink : $n = K\, M^{-a}$ où K et a sont des constantes pour un système polymère-solvant à une température fixée.

## EXEMPLE 4 : CINETIQUE DE RELARGAGE - PRINCIPAUX PARAMETRES AJUSTABLES

### 1) choix du polymère

Le polymère de composition 50/50 se dégrade plus rapidement que des copolymères ayant d'autres proportions d'acide lactique et glycolique. (Voir référence : Miller R.A., Brady J.M. et Cutright D.E. - J. Biomed. Mater. res., 11, 711, 1977).

La viscosité intrinsèque du PLGA qui est d'ailleur fonction principalement de la taille des polymères, intervient également dans la cinétique de relargage. Ainsi, pour des microsphères de calcitonine préparées avec des PLGA 50/50 de différentes viscosités, un relargage in vitro de la calcitonine montre que plus la viscositié intrinsèque du PLGA est élevée (plus la masse moléculaire du PLGA est élevée) plus lent est le relargage de la calcitonine dans le milieu extérieur. Ainsi, pour une incorporation en PLGA 50/50 de viscosité 0,4 (PM mesuré par GPC : 36000), 0,5 (50000) ou 0,7 (91000), le pourcentage de calcitonine relarguée in vitro après 14 jours est de 79,5, 75,5 ou 43,2 % respectivement.

2) Taille des microsphères

Exemple 4-a : Influence de la taille des microsphères sur le relargage du principe actif

Des microsphères de calcitonine ont été préparées selon le protocole suivant.

Matériel :

Phase polymère :
- polymère PLGA 50/50 n = 0,8     : 2,5 g
- solvant du PLGA : $CH_2Cl_2$     : 20,0 g
- peptide : CTs (calcitonine de saumon)     : 10,0 mg
  taux de charge de 0,4 %/PLGA
- peptide marqué $I^{125}$ : CTs $I^{125}$     : 2,39 $10^6$ cpm
- ballast : lactose     :5,0 mg
  taux de charge de 0,2 %/PLGA
- "tiers solvant" : DMA     : 2,5 ml

Phase aqueuse :
- tampon phosphate 10 mM pH 8 avec 4 % de gélatine (=émulsifiant)     : 75,0 ml

Méthode :

- Le peptide, le traceur et le ballast sont solubilisés dans le DMA.
- Ils sont ensuite ajoutés au polymère en solution dans le $CH_2Cl_2$ pour former une phase homogène, la phase polymère.
- Emulsion : la phase aqueuse est alors superposée à la phase polymère et mélangée à la phase polymère à l'aide d'une hélice tournant à 1500 tours/min pendant une minute.
- Evaporation : une agitation de 500 tours/min est maintenue pendant ± 12 heures, à température ambiante, avec aspiration d'air à la surface du mélange émulsionné.
- L'émulsion est ensuite diluée 2 fois dans de l'eau et agitée pendant 1 heure, à 37°C.
- Les microsphères sont récupérées dans l'eau sur des tamis successifs de mailles de plus en plus fines 200-100-50-20 micromètres.
- Elles sont ensuite séchées pendant 24 heures à l'étuve sous vide, à température ambiante.
- Puis, elles sont irradiées à 2,5 Mega Rad.

Résultats :

- Granulométrie des microsphères obtenues :

| taille (x) en microns | $25 < x < 50\,\mu m$ | $50 < x < 100\mu m$ | $100 < x < 200\mu m$ | $x > 200\,\mu m$ |
|---|---|---|---|---|
| Pourcentage en poids des microsphères | 7,5 % | 39,3 % | 46,2 % | 0,7 % |

- Le taux d'incorporation de calcitonine dans les microsphères : il est mesuré grâce au traceur (CTs $I^{125}$) introduit dans la préparation et en tenant compte du rendement en poids des microsphères par rapport au poids du polymère engagé. On trouve un total de 85,1 % de CTs incorporée.
- Relargage "in vitro" de la calcitonine :
200 mg de microsphères sont placées dans 10 ml de tampon phosphate pH 7 10 mM contenant 0,5 % de BSA à 37°C dans un bain oscillant.

Tableau représentant les pourcentages cumulatifs de calcitonine libérée au cours du temps :

| Taille des microsphères | 25-50 μm | 50-100 μm | 100-200 μm |
|---|---|---|---|
| Après : 1 jour | 11,2 % | 3,2 % | 2,8 % |
| 3 jours | 16,0 % | 4,5 % | 4,0 % |
| 4 jours | 16,5 % | 5,0 % | 4,7 % |
| 7 jours | 19,8 % | 5,5 % | 4,9 % |
| 10 jours | 25,2 % | 7,4 % | 6,4 % |
| 14 jours | 31,4 % | 7,7 % | 7,0 % |
| 25 jours | 73,0 % | 31,6 % | 31,0 % |
| 28 jours | 100,0 % | 40,2 % | 39,0 % |
| 35 jours | - | 64,5 % | 56,0 % |
| 40 jours | | 89,0 % | 79,0 % |
| 42 jours | | 100,0 % | 89,0 % |
| 45 jours | | - | 100,0 % |

<u>Exemple 4b</u> : Paramètres influençant la taille microsphères

<u>Exemple 4b-I</u> : la vitesse d'émulsion
- PLGA 50/50 : 2,5 g mis en solution dans $CH_2Cl_2$ : 20 g
  Calcitonine de saumon : 5 mg dissoute dans 2,5 ml de DMA, puis ajoutée à la phase polymère.
- Phase aqueuse : 75 ml de gélatine 5 % (poids/volume) dans l'eau.
- Les 2 phases sont émulsionnées
  . soit par agitation d'une hélice à 2000 tours/minute pendant 1 minute,
  . soit par agiation d'une hélice à 500 tours/minute pendant 1 minute.
- L'évaporation du $CH_2Cl_2$ et le passage dans l'eau du DMA se font sous une agitation continue de 500 tours/minute.
- Après plusieurs lavages à l'eau, un échantillon de microsphères est analysé dans le "Fristch particle sizer" (Analysette 22).

<u>Résultats</u>

| Pourcentage des microsphères (en poids) ayant une taille inférieure à | Emulsion à 2000 t/min Taille des microsphères(μm) | emulsion à 500 t/min Taille des microsphères( μm) |
|---|---|---|
| 10 % < | 16,4 | 78,9 |
| 30 % < | 35,2 | 164,0 |
| 50 % < | 52,3 | 212,0 |
| 70 % < | 73,0 | 271,0 |
| 90 % < | 126,0 | 351,0 |

Exemple 4b-II : La concentration de l'émulsifiant
- PLGA 50/50 : 2,5 g mis en solution dans $CH_2Cl_2$ : 20 g
  Calcitonine de saumon : 5 mg dissoute dans 2,5 ml de DMA, puis ajoutée à la phase polymère.
- Phase aqueuse :
  . soit : 75 ml gélatine 5 % (poids/volume) dans l'eau,
  . soit : 75 ml gélatine 1 % (poids/volume) dans l'eau.
- Les deux phases sont émulsionnées par agitation d'une hélice à 2000 tour/minute pendant 1 minute.
- L'évaporation du $CH_2Cl_2$ et le passage dans l'eau du DMA se font sous une agitation de 500 tours/minute avec une hélice.
- Après plusieurs lavages à l'eau, les microsphères sont analysées dans le "Fritsch Particle Sizer" (Analysette 22).

Résultats

| Pourcentage des microsphères (en poids) ayant une taille inférieure à | Emulsifiant : gélatine 5 % Taille des microsphères( μm) | Emulsifiant : gélatine 1 % Taille des microsphères( μm) |
|---|---|---|
| 10 % < | 16,4 | 37,0 |
| 30 % < | 35,2 | 73,6 |
| 50 % < | 52,3 | 104,0 |
| 70 % < | 73,0 | 140,0 |
| 90 % < | 126,0 | 215,0 |

3) Choix du ballast

La présente ou la concentration de ballast dans les microsphères ainsi que la nature du ballast peut influencer la cinétique de relargage de la calcitonine comme le montre l'expérience suivante :

Matériel :

Phase polymère :
- PLGA 50/50 : 2,5 g dissout dans
  $CH_2Cl_2$ : 20,0 g
- Calcitonine de saumon : 10 mg dissoute dans :
  DMA : 2,5 ml
- Ballast :

| A | B | C |
|---|---|---|
| Sans | Lactose 0,2 % | Gélatine 0,2 % |

Phase aqueuse :
- Tampon phosphate 10 mM pH8
75 ml avec 4 % de gélatine (poids/volume)

Méthode :

- Les deux phases sont émulsionnées par agitation d'une hélice à 1500 tours/minute pendant une minute.

- Une agitation de 500 tours/minute est ensuite maintenue jusqu'à évaporation complète du $CH_2Cl_2$ et passage dans la phase aqueuse du DMA.
- Après plusieurs lavages à l'eau, les microsphères sont récupérées sur des tamis et séchées sous vide.

Résultats :

Relargage "in vitro" obtenu dans les mêmes conditions qu'au point 2) de l'exemple 3.

| | Pourcentage cumulatif de CTs relarguée | | |
|---|---|---|---|
| | A<br>sans ballast | B<br>(+ 0,2 % lactose) | C<br>(+ 0,2 % gélatine) |
| Après : 1 jour | 2,0 % | 2,8 | 6 |
| 2 jours | 2,2 % | 4,0 | 8 |
| 7 jours | 3,4 % | 5,0 | 11 |
| 12 jours | 4,3 % | 7,4 | 15 |
| 20 jours | 16,0 % | 22,0 | 36 |
| 25 jours | 25,0 % | 31,0 | 46 |
| 30 jours | 32,0 % | 41,0 | 59 |
| 35 jours | 39,0 % | 56,0 | 83 |
| 37 jours | 44,0 % | 66,0 | 88 |
| 40 jours | 59,0 % | 79,0 | 100 |
| 42 jours | 69,0 | 100,0 | - |
| 48 jours | 100,0 | - | - |

4) Taux de charge en peptide

Matériel

Phase polymère :
- PLGA 50/50 : 0,5 g dissous dans :
  $CH_2Cl_2$ : 10 g
- DMA : 3 ml dans lequel est dissoute la calcitonine de saumon en quantité suivante :

| A | B |
|---|---|
| 10 mg<br>taux de charge de 2 % | 25 mg<br>taux de charge de 5 % |

- Ballast : sans
      Phase aqueuse :

- Tampon phosphate 10 mM pH 8 avec 4 % de gélatine (poids/volume) 75 ml.

Méthode

Relargage "in vitro" obtenu dans les mêmes conditions qu'au point 2) de l'exemple 3.

| Taux de charge en calcitonine de saumon | Pourcentage cumulatif de CTs relarguée | |
|---|---|---|
| | A 2 % | B 5 % |
| Après : 1 jour | 3,0 % | 6,5 % |
| 4 jours | 4,8 % | 13,0 % |
| 6 jours | 5,7 % | 16,0 % |
| 10 jours | 7,4 % | 18,5 % |
| 15 jours | 8,6 % | 23,0 % |
| 21 jours | 9,7 % | 27,0 % |

**EXEMPLE 5 : DIMINUTION DU TAUX RESIDUEL EN SOLVANT TOXIQUE DES MICROSPHERES**

Matériel

- PLGA 50/50 : 1 g dissous dans 7 g de $CH_2Cl_2$.
- Calcitonine de saumon : 20 mg dissoute dans 2 ml de DMA, puis ajoutée à la phase polymère.
- Phase aqueuse :
    a. * soit : 100 ml de gélatine 5 % (poids/volume) dans du tampon phosphate 10 mM ph8.
    b. * soit : un mélange de 80 ml de gélatine 5 % (poids/volume) dans du tampon phosphate 10 mM ph8 + 20 ml d'éthanol.

Méthode

- Les deux phases sont émulsionnées à l'aide d'une hélice tournant à 1.500 tours/minute, pendant 1 minute.
- Une agitation de 500 tours/minute, pendant 12 heures, à 37° C, avec aspiration d'air permet l'évaporation du $CH_2Cl_2$ et le passage du DMA vers la phase aqueuse.
- Les microsphères sont récupérées sur des tamis successifs de 200 et de 25 microns.
- Puis, elles sont séchées pendant 24 heures à l'étuve sous vide, à température ambiante, et ensuite irradiées à 2,5 M Rad.

Résultats

- Les taux en dichlorométhane résiduel dans les microsphères sèches (fraction de 25 à 200 micromètres) sont déterminés par chromatographie en phase aqueuse.

Ces taux sont exprimés en pourcentage de solvant par rapport au poids sec des microsphères.

| Méthode de préparation | Taux résiduel en $CH_2Cl_2$ |
|---|---|
| a. sans éthanol dans la phase aqueuse | 1,36 % |
| b. avec 20 % d'éthanol dans la phase aqueuse | 0,14 % |

## EXEMPLE 6 : ADAPTATION DE LA TECHNIQUE A D'AUTRES POLYPEPTIDES

L'application de la technique est conditionnée par la solubilité des polypeptides dans le tiers solvant. Cette propriété est commune à de nombreux polypeptides, comme nous l'avons montré pour la calcitonine.

Exemple :

- la LHRH ("Luteinizing Hormone Releasing Hormone") est soluble à au moins 50 mg/ml dans le DMA.
- l'HGH ("Human Growth Hormone") est soluble à 2,6 mg/ml de DMA.
- Afin d'augmenter la solubilité de certains polypeptides moins solubles dans le "tiers solvant", il est possible d'ajouter une solution aqueuse du peptide au "tiers solvant" qui est bien entendu miscible à l'eau.
- C'est ainsi que les peptides suivants ont pu être incorporés dans les microsphères de PLGA par la technique selon l'invention : calcitonine, hGh, bGH, ANF, somatostatine, LHRH, insuline, somatomédine C, ACTH, ADH, TPA, ainsi que différentes cytokines : érythropoiétine, GMCSF, GCSF, IL3, IL1, IL6, IL2, IL4, TNF, interféron alpha, interféron bêta, TGF bêta, PDGF, EGF.
- Le tableau ci-après reprend les taux d'incorporation obtenus pour différents peptides en utilisant la procédure "standard" décrite ci-dessous selon l'invention sans optimisation individuelle pour chaque peptide. Les valeurs représentent donc un taux minimal d'incorporation. Le protocole standard utilisé est le suivant :

Matériel

- PLGA 50/50 : 2,5 g dissous dans 20 g de $CH_2Cl_2$.
Tiers solvant : DMA : 2,5 ml dans lequel est dissous le principe actif.
Phase aqueuse :
75 ml de gélatine 4 % (poids/volume) dans du tampon phosphate 10 mM pH 8.

Méthode

La phase polymère dans laquelle le principe actif est dissout grâce au tiers solvant, est émulsionnée avec la phase aqueuse par une hélice agitée à 1500 tours/minute et ce, pendant une minute.
- Une agitation de 500 tours/minute est maintenue à température ambiante avec aspiration d'air afin de faciliter l'évaporation de $CH_2Cl_2$ et le passage dans l'eau du tiers solvant.
- Les microsphères sont ensuite récupérées sur un tamis après avoir été lavées abondamment à l'eau.
- Puis elles sont séchées à température ambiante, sous vide.

| Principe actif incorporé dans les microsphères | Pourcentage du peptide incorporé |
|---|---|
| hGH (hormone de croissance humaine) | 77,0 % |
| Somatomédine C (IGF-1) | 52,0 % |
| TPA (Tissue Plasminogen Activator) | 96,0 % |
| Insuline | 98,0 % |
| IL4 | 77,5 % |
| IL6 | 43,8 % |
| Erythropoïétine | 40,0 % |
| TNF | 66,4 % |
| Calcitonine de saumon | 92,4 % |
| Calcitonine humaine | 85,0 % |
| IL2 | 78,5 % |
| GRH | 84,0 % |
| LHRH | 75,0 % |
| GMCSF | 59,0 % |
| PTH (1-34) | 54,0 % |
| Somatostatine | 82,0 % |
| ANP (ou ANF) | 72,0 % |
| Interféron alpha | 77,0 % |

## Revendications

**Revendications pour les Etats contractants suivants : AT, BE, CH, LI, DE, DK, FR, GB, IT, NL, LU, SE**

1. Procédé de préparation de microparticules de type microsphères d'une substance hydrosoluble et d'un polymère biocompatible et biodégradable contrôlant la cinétique de libération de ladite substance constituée d'une matrice dudit polymère au sein de laquelle est régulièrement dispersée ladite substance hydrosoluble, caractérisé en ce que

   a) ledit polymère est dissous dans un premier solvant organique volatile non miscible à l'eau,

   b) ladite substance hydrosoluble est dissoute séparément dans un second solvant qui est miscible audit premier solvant, est un solvant du polymère, et miscible à l'eau, la proportion en second solvant par rapport au premier solvant allant de 5/95 à 70/30 en volume

   c) on mélange la solution de ladite substance et la solution dudit polymère,

   d) on obtient une phase organique du polymère et de ladite substance qui est ensuite émulsionnée dans un milieu non miscible dispersant, consistant en une phase aqueuse contenant un agent émulsifiant,

   e) les deux solvants sont ensuite éliminés des microsphères en formation sous agitation, le premier solvant étant éliminé par évaporation, le second solvant ainsi qu'une partie du premier solvant qui lui est miscible étant éliminés par passage vers la phase aqueuse par un mécanisme de séparation de phase,

   f) après élimination des solvants, on récupère les microsphères formées éventuellement après lavage à l'eau et tamisage.

2. Procédé selon la revendication 1, caractérisé en ce que la phase dispersante aqueuse comporte un troisième solvant qui est miscible à l'eau et miscible audit premier solvant.

3. Procédé selon la revendication 1 ou 2, caractérisé en ce que ledit second solvant est choisi parmi le DMA, THF, DMSO, DMF ou dioxane.

4. Procédé selon l'une des revendications 1, 2 ou 3, caractérisé en ce que ledit premier solvant est le dichlorométhane et ledit second solvant est le diméthylacétamide.

5. Procédé selon la revendication 2 ou 3, caractérisé en ce que ledit troisième solvant est l'éthanol.

6. Procédé selon l'une des revendications 1 à 5, caractérisé en ce que ladite substance hydrosoluble est un polypeptide ou un de ses sels pharmaceutiquement acceptables.

7. Procédé selon l'une des revendications précédentes, caractérisé en ce que le polymère est un copolymère d'acide lactique et glycolique.

8. Procédé selon l'une des revendications précédentes, caractérisé en ce que le polymère est un copolymère d'acide lactique est glycolique qui comprend une proportion entre les unités d'acide lactique et glycolique respectivement de 40:60 à 75:25.

9. Procédé selon l'une des revendications précédentes, caractérisé en ce que la concentration en agent émulsifiant est comprise entre 0,1 et 10 % (poids/volume) dans la phase aqueuse.

10. Microparticules de type microsphères prouvant être obtenues selon le procédé de l'une des revendications précédentes.

11. Microparticules de type microsphères constituées d'une matrice d'un polymère biocompatible et biodégradable soluble dans un solvant organique non miscible dans l'eau, au sein de laquelle est régulièrement répartie une substance hydrosoluble, caractérisées en ce que le taux résiduel en solvant est inférieur à 1,5% en poids.

12. Microparticules de type microsphères selon la revendication 10, caractérisées en ce que le taux résiduel en solvant est inférieur à 0,5 %, de préférence inférieur à 0,2 % en poids.

13. Microsphère selon l'une des revendications 9 à 11, caractérisée en ce que la substance hydrosoluble est un polypeptide.

14. Microsphère selon l'une des revendications précédentes, caractérisée en ce que le polymère est un copolymère d'acide lactique et d'acide glycolique, le polypeptide est la calcitonine ou ses analogues et dérivés.

15. Microsphère selon l'une des revendications précédentes, caractérisée en ce qu'elles ont une taille comprise entre 25 et 200 $\mu$m.

16. Microsphère selon l'une des revendications précédentes, caractérisée en ce que le taux de charge en substance hydrosoluble est compris entre 0,01 et 40 %, de préférence entre 1 et 10 % en poids de la microsphère.

**Revendications pour les Etats contractants suivants : ES, GR**

1. Procédé de préparation de microparticules de type microsphères d'une substance hydrosoluble et d'un polymère biocompatible et biodégradable contrôlant la cinétique de libération de ladite substance constituée d'une matrice dudit polymère au sein de laquelle est régulièrement dispersée ladite substance hydrosoluble, caractérisé en ce que
   a) ledit polymère est dissous dans un premier solvant organique volatile non miscible à l'eau,
   b) ladite substance hydrosoluble est dissoute séparément dans un second solvant qui est miscible audit premier solvant, est un solvant du polymère, et miscible à l'eau, la proportion en second solvant par rapport au premier solvant allant de 5/95 à 70/30 en volume
   c) on mélange la solution de ladite substance et la solution dudit polymère,
   d) on obtient une phase organique du polymère et de ladite substance qui est ensuite émulsionnée dans un milieu non miscible dispersant, consistant en une phase aqueuse contenant un agent émulsifiant,
   e) les deux solvants sont ensuite éliminés des microsphères en formation sous agitation, le premier solvant étant éliminé par évaporation, le second solvant ainsi qu'une partie du premier solvant qui lui est miscible étant éliminés par passage vers la phase aqueuse par un mécanisme de séparation de phase,

f) après élimination des solvants, on récupère les microsphères formées éventuellement après lavage à l'eau et tamisage.

**2.** Procédé selon la revendication 1, caractérisé en ce que la phase dispersante aqueuse comporte un troisième solvant qui est miscible à l'eau et miscible audit premier solvant.

**3.** Procédé selon la revendication 1 ou 2, caractérisé en ce que ledit second solvant est choisi parmi le DMA, THF, DMSO, DMF ou dioxane.

**4.** Procédé selon l'une des revendications 1, 2 ou 3, caractérisé en ce que ledit premier solvant est le dichlorométhane et ledit second solvant est le diméthylacétamide.

**5.** Procédé selon la revendication 2 ou 3, caractérisé en ce que ledit troisième solvant est l'éthanol.

**6.** Procédé selon l'une des revendications 1 à 5, caractérisé en ce que ladite substance hydrosoluble est un polypeptide ou un de ses sels pharmaceutiquement acceptables.

**7.** Procédé selon l'une des revendications précédentes, caractérisé en ce que le polymère est un copolymère d'acide lactique et glycolique.

**8.** Procédé selon l'une des revendications précédentes, caractérisé en ce que le polymère est un copolymère d'acide lactique est glycolique qui comprend une proportion entre les unités d'acide lactique et glycolique respectivement de 40:60 à 75:25.

**9.** Procédé selon l'une des revendications précédentes, caractérisé en ce que la concentration en agent émulsifiant est comprise entre 0,1 et 10 % (poids/volume) dans la phase aqueuse.

## Patentansprüche

### Patentansprüche für folgende Vertragsstaaten : AT, BE, CH, LI, DE, DK, FR, GB, IT, LU, NL, SE

**1.** Verfahren zur Herstellung von Mikroteilchen vom Mikrokugel-Typ aus einer wasserlöslichen Substanz und einem biokompatiblen und biologisch abbaubaren Polymer, das die Freisetzungskinetik der genannten Substanz steuert (kontrolliert), die besteht aus einer Matrix des genannten Polymers, in der die genannte wasserlösliche Substanz gleichmäßig dispergiert ist, dadurch gekennzeichnet, daß

a) das genannte Polymer in einem ersten, mit Wasser nicht mischbaren flüchtigen organischen Lösungsmittel gelöst wird,

b) die genannte wasserlösliche Substanz getrennt in einem zweiten Lösungsmittel gelöst wird, das mit dem ersten Lösungsmittel mischbar ist, das ein Lösungsmittel für das Polymer ist und mit Wasser mischbar ist, wobei das Volumenverhältnis zwischen dem zweiten Lösungsmittel und dem ersten Lösungsmittel 5/95 bis 70/30 beträgt,

c) die Lösung der genannten Substanz mit der Lösung des genannten Polymers gemischt wird, wobei man

d) eine organische Phase des Polymers und der genannten Substanz erhält, die anschließend in einem nicht mischbaren Dispergiermittel-Medium emulgiert wird, das besteht aus einer wäßrigen Phase, die ein Emulgiermittel enthält,

e) die beiden Lösungsmittel anschließend aus den sich bildenden Mikrokugeln unter Rühren eliminiert werden, wobei das erste Lösungsmittel durch Verdampfen eliminiert wird, während das zweite Lösungsmittel sowie ein Teil des ersten Lösungsmittels, das damit mischbar ist, eliminiert werden durch Überführung in die wäßrige Phase nach einem Phasentrennungsmechanismus und

f) nach der Eliminierung der Lösungsmittel die gebildeten Mikrokugeln, gegebenenfalls nach dem Waschen mit Wasser und nach dem Sieben, gewonnen (abgetrennt) werden.

**2.** Verfahren nach Anspruch 1, dadurch gekennzeichnet, daß die dispergierende wäßrige Phase ein drittes Lösungsmittel enthält, das mit Wasser und mit dem ersten Lösungsmittel mischbar ist.

**3.** Verfahren nach Anspruch 1 oder 2, dadurch gekennzeichnet, daß das genannte zweite Lösungsmittel ausgewählt wird aus DMA, THF, DMSO, DMF oder Dioxan.

4. Verfahren nach einem der Ansprüche 1, 2 oder 3, dadurch gekennzeichnet, daß das genannte erste Lösungsmittel Dichlormethan ist und daß das genannte zweite Lösungsmittel Dimethylacetamid ist.

5. Verfahren nach Anspruch 2 oder 3, dadurch gekennzeichnet, daß das genannte dritte Lösungsmittel Ethanol ist.

6. Verfahren nach einem der Ansprüche 1 bis 5, dadurch gekennzeichnet, daß die genannte wasserlösliche Substanz ein Polypeptid oder eines seiner pharmazeutisch akzeptablen Salze ist.

7. Verfahren nach einem der vorhergehenden Ansprüche, dadurch gekennzeichnet, daß das Polymer ein Copolymer von Milchsäure und Glycolsäure ist.

8. Verfahren nach einem der vorhergehenden Ansprüche, dadurch gekennzeichnet, daß das Polymer ein Copolymer von Milchsäure und Glycolsäure ist, das die jeweiligen Milchsäure- und Glycolsäureeinheiten in einem Mengenverhältnis von 40:60 bis 75:25 enthält.

9. Verfahren nach einem der vorhergehenden Ansprüche, dadurch gekennzeichnet, daß die Konzentration an Emulgiermittel in der wäßrigen Phase zwischen 0,1 und 10 Gew./Vol.-% liegt.

10. Mikroteilchen vom Mikrokugel-Typ, wie sie nach dem Verfahren nach einem der vorhergehenden Ansprüche erhältlich sind.

11. Mikroteilchen vom Mikrokugel-Typ, bestehend aus einer Matrix eines biokompatiblen und biologisch abbaubaren Polymers, das in einem mit Wasser nicht mischbaren organischen Lösungsmittel löslich ist, in der eine wasserlösliche Substanz gleichmäßig verteilt ist, dadurch gekennzeichnet, daß der Restgehalt an Lösungsmittel unter 1,5 Gew.-% liegt.

12. Mikroteilchen vom Mikrokugel-Typ nach Anspruch 10, dadurch gekennzeichnet, daß der Restgehalt an Lösungsmittel unterhalb 0,5 Gew.-%, vorzugsweise unterhalb 0,2 Gew.-%, liegt.

13. Mikrokugel nach einem der Ansprüche 9 bis 11, dadurch gekennzeichnet, daß die wasserlösliche Substanz ein Polypeptid ist.

14. Mikrokugel nach einem der vorhergehenden Ansprüche, dadurch gekennzeichnet, daß das Polymer ein Copolymer von Milchsäure und Glycolsäure ist und daß das Polypeptid das Calcitonin oder ein Analogen oder ein Derivat davon ist.

15. Mikrokugel nach einem der vorhergehenden Ansprüche, dadurch gekennzeichnet, daß sie eine Größe zwischen 25 und 200 μm hat.

16. Mikrokugel nach einem der vorhergehenden Ansprüche, dadurch gekennzeichnet, daß der Gehalt der Beschickung an wasserlöslicher Substanz zwischen 0,01 und 40 Gew.-%, vorzugsweise zwischen 1 und 10 Gew.-%, bezogen auf das Gewicht der Mikrokugel, liegt.

**Patentansprüche für folgende Vertragsstaaten : ES, GR**

1. Verfahren zur Herstellung von Mikroteilchen vom Mikrokugel-Typ aus einer wasserlöslichen Substanz und einem biokompatiblen und biologisch abbaubaren Polymer, das die Freisetzungskinetik der genannten Substanz steuert (kontrolliert), die besteht aus einer Matrix des genannten Polymers, in der die genannte wasserlösliche Substanz gleichmäßig dispergiert ist, dadurch gekennzeichnet, daß

a) das genannte Polymer in einem ersten, mit Wasser nicht mischbaren flüchtigen organischen Lösungsmittel gelöst wird,
b) die genannte wasserlösliche Substanz getrennt in einem zweiten Lösungsmittel gelöst wird, das mit dem ersten Lösungsmittel mischbar ist, das ein Lösungsmittel für das Polymer ist und mit Wasser mischbar ist, wobei das Volumenverhältnis zwischen dem zweiten Lösungsmittel und dem ersten Lösungsmittel 5/95 bis 70/30 beträgt,
c) die Lösung der genannten Substanz mit der Lösung des genannten Polymers gemischt wird, wobei man
d) eine organische Phase des Polymers und der genannten Substanz erhält, die anschließend in einem nicht mischbaren Dispergiermittel-Medium emulgiert wird, das besteht aus einer wäßrigen Phase, die ein Emulgiermittel enthält,

e) die beiden Lösungsmittel anschließend aus den sich bildenden Mikrokugeln unter Rühren eliminiert werden, wobei das erste Lösungsmittel durch Verdampfen eliminiert wird, während das zweite Lösungsmittel sowie ein Teil des ersten Lösungsmittels, das damit mischbar ist, eliminiert werden durch Überführung in die wäßrige Phase nach einem Phasentrennungsmechanismus und

f) nach der Eliminierung der Lösungsmittel die gebildeten Mikrokugeln, gegebenenfalls nach dem Waschen mit Wasser und nach dem Sieben, gewonnen (abgetrennt) werden.

2. Verfahren nach Anspruch 1, dadurch gekennzeichnet, daß die dispergierende wäßrige Phase ein drittes Lösungsmittel enthält, das mit Wasser und mit dem ersten Lösungsmittel mischbar ist.

3. Verfahren nach Anspruch 1 oder 2, dadurch gekennzeichnet, daß das genannte zweite Lösungsmittel ausgewählt wird aus DMA, THF, DMSO, DMF oder Dioxan.

4. Verfahren nach einem der Ansprüche 1, 2 oder 3, dadurch gekennzeichnet, daß das genannte erste Lösungsmittel Dichlormethan ist und daß das genannte zweite Lösungsmittel Dimethylacetamid ist.

5. Verfahren nach Anspruch 2 oder 3, dadurch gekennzeichnet, daß das genannte dritte Lösungsmittel Ethanol ist.

6. Verfahren nach einem der Ansprüche 1 bis 5, dadurch gekennzeichnet, daß die genannte wasserlösliche Substanz ein Polypeptid oder eines seiner pharmazeutisch akzeptablen Salze ist.

7. Verfahren nach einem der vorhergehenden Ansprüche, dadurch gekennzeichnet, daß das Polymer ein Copolymer von Milchsäure und Glycolsäure ist.

8. Verfahren nach einem der vorhergehenden Ansprüche, dadurch gekennzeichnet, daß das Polymer ein Copolymer von Milchsäure und Glycolsäure ist, das die jeweiligen Milchsäure- und Glycolsäureeinheiten in einem Mengenverhältnis von 40:60 bis 75:25 enthält.

9. Verfahren nach einem der vorhergehenden Ansprüche, dadurch gekennzeichnet, daß die Konzentration an Emulgiermittel in der wäßrigen Phase zwischen 0,1 und 10 Gew./Vol.-% liegt.

## Claims

### Claims for the following Contracting States : AT, BE, CH, LI, DE, DK, FR, GB, IT, LI, LU, NL, SE

1. Process for preparing microparticles of the microsphere type of a water-soluble substance and a biocompatible and biodegradable polymer controlling the kinetics of release of the said substance consisting of a matrix of the said polymer within which the said water-soluble substance is regularly dispersed, characterized in that

a) the said polymer is dissolved in a first volatile organic solvent immiscible with water,

b) the said water-soluble substance is separately dissolved in a second solvent which is miscible with the said first solvent, is a solvent for the polymer, and miscible with water, the proportion of second solvent to first solvent ranging from 5/95 to 70/30 by volume,

c) the solution of the said substance and the solution of the said polymer are mixed,

d) an organic phase of the polymer and of the said substance is obtained which is then emulsified in an immiscible dispersant medium consisting of an aqueous phase containing an emulsifying agent,

e) the two solvents are then removed from the microspheres being formed with stirring, the first solvent being removed by evaporation, the second solvent as well as part of the first solvent which is miscible therewith being removed by passage towards the aqueous phase by a mechanism of phase separation,

f) after removal of the solvents, the microspheres formed are recovered, optionally after washing in water and sieving.

2. Process according to claim 1, characterized in that the aqueous dispersant phase comprises a third solvent which is miscible with water and miscible with the said first solvent.

3. Process according to Claim 1 or 2, characterized in that the said second solvent is chosen from DMA, THF, DMSO, DMF or dioxane.

4. Process according to one of Claims 1, 2 or 3, characterized in that the said first solvent is dichloromethane and the said second solvent is dimethylacetamide.

5. Process according to Claim 2, characterized in that the said third solvent is ethanol.

6. Process according to one of Claims 1 to 5, characterized in that the said water-soluble substance is a polypeptide or one of its pharmaceutically acceptable salts.

7. Process according to one of the preceding claims, characterized in that the polymer is a lactic acid and glycolic acid copolymer.

8. Process according to one of the preceding claims, characterized in that the polymer is a lactic acid and glycolic acid copolymer which comprises a proportion between the lactic acid and glycolic acid units of 40:60 to 75:25 respectively.

9. Process according to one of the preceding claims, characterized in that the concentration of emulsifying agent in the aqueous phase is between 0.1 and 10% (weight/volume).

10. Microparticles of the microsphere type which can be obtained according to the process of one of the preceding claims.

11. Microparticles of the microsphere type consisting of a matrix of a biocompatible and biodegradable polymer which is soluble in an organic solvent which is immiscible in water, within which a water-soluble substance is regularly distributed, characterized in that the residual level of solvent is lower than 1.5% by weight.

12. Microparticles of the microsphere type according to Claim 10, characterized in that the residual level of solvent is lower than 0.5%, preferably lower than 0.2% by weight.

13. Microsphere according to one of Claims 9 to 11, characterized in that the water-soluble substance is a polypeptide.

14. Microsphere according to one of the preceding claims, characterized in that the polymer is a lactic acid and glycolic acid copolymer, and the polypeptide is calcitonin or its analogues and derivatives.

15. Microsphere according to one of the preceding claims, characterized in that they have a size of between 25 and 200 μm.

16. Microsphere according to one of the preceding claims, characterized in that the level of charge of water-soluble substance is between 0.01 and 40%, preferably between 1 and 10% by weight of the microsphere.

**Claims for the following Contracting States : ES, GR**

1. Process for preparing microparticles of the microsphere type of a water-soluble substance and a biocompatible and biodegradable polymer controlling the kinetics of release of the said substance consisting of a matrix of the said polymer within which the said water-soluble substance is regularly dispersed, characterized in that
a) the said polymer is dissolved in a first volatile organic solvent immiscible with water,
b) the said water-soluble substance is separately dissolved in a second solvent which is miscible with the said first solvent, is a solvent for the polymer, and miscible with water, the proportion of second solvent to first solvent ranging from 5/95 to 70/30 by volume,
c) the solution of the said substance and the solution of the said polymer are mixed,
d) an organic phase of the polymer and of the said substance is obtained which is then emulsified in an immiscible dispersant medium consisting of an aqueous phase containing an emulsifying agent,
e) the two solvents are then removed from the microspheres being formed with stirring, the first solvent being removed by evaporation, the second solvent as well as part of the first solvent which is miscible therewith being removed by passage towards the aqueous phase by a mechanism of phase separation,
f) after removal of the solvents, the microspheres formed are recovered, optionally after washing in water and sieving.

2. Process according to Claim 1, characterized in that the aqueous dispersant phase comprises a third solvent which is miscible with water and miscible with the said first solvent.

3. Process according to Claim 1 or 2, characterized in that the said second solvent is chosen from DMA, THF, DMSO, DMF or dioxane.

4. Process according to one of Claim 1, 2 or 3, characterized in that the said first solvent is dichloromethane and the said second solvent is dimethylacetamide.

5. Process according to Claim 2 or 3, characterized in that the said third solvent is ethanol.

6. Process according to one of Claims 1 to 5, characterized in that the said water-soluble substance is a polypeptide or one of its pharmaceutically acceptable salts.

7. Process according to one of the preceding claims, characterized in that the polymer is a lactic acid and glycolic acid copolymer.

8. Process according to one of the preceding claims, characterized in that the polymer is a lactic acid and glycolic acid copolymer which comprises a proportion between the lactic acid and glycolic acid units of 40:60 to 75:25 respectively.

9. Process according to one of the preceding claims, characterized in that the concentration of emulsifying agent in the aqueous phase is between 0.1 and 10% (weight/volume).